# EUROPEAN PATENT APPLICATION

(11) **EP 4 458 797 A1**
(43) Date of publication of application: **06.11.2024**
(21) Application number: 22914136.1
(22) Date of filing: 08.12.2022
(51) Int. Cl.: C07C 39/04, C07C 49/403, C07B 33/00

(54) **VISIBLE-LIGHT-MEDIATED ONE-STEP METHOD FOR PREPARING PHENOL AND CYCLOHEXANONE FROM CYCLOHEXYLBENZENE**

(30) Priority: 27.12.2021 CN 202111613495
(71) Applicant: Fudan University, Shanghai 200433 (CN)
(72) Inventor: MA, Shengming, Shanghai 200433 (CN); YU, Hao, Shanghai 200433 (CN); LIU, Qi, Shanghai 200433 (CN); SHI, Zhangjie, Shanghai 200438 (CN)
(74) Representative: Barker Brettell LLP
(86) International application number: PCT/CN2022/137617
(87) International publication number: WO 2023/124881

(57) **Abstract**

The present invention relates to a visible-light-mediated one-step method for preparing phenol and cyclohexanone from cyclohexylbenzene (CHB). The method specifically comprises: under the irradiation of visible light, and with a hydrogen bromide solution as a catalyst and oxygen as an oxidizing agent, directly oxidizing cyclohexylbenzene in an organic solvent to break a carbon-carbon bond, so as to generate phenol and cyclohexanone. The method avoids such a process in a cyclohexylbenzene method in the prior art that cyclohexylbenzene needs to be first oxidized to obtain cyclohexylbenzene-1-hydroperoxide (1-CHBHP), then the mixture of the oxidation reaction products is treated, and then a cyclohexylbenzene peroxide is decomposed in an acidic condition to obtain phenol and cyclohexanone, and also avoids potential risks caused by the accumulation of peroxides. In addition, the method further has a great number of advantages of the operation being simple, reagents being easily available, reaction conditions being mild, easy control being achievable, the reaction being able to be scaled-up, a good selectivity being obtained, etc.

## Description

### TECHNICAL FIELD

The invention belongs to the field of organic synthesis application technology, and relates to a method for preparing phenol and cyclohexanone in one step from cyclohexylbenzene mediated by visible light.

### BACKGROUND OF THE INVENTION

Both phenol and cyclohexanone are important basic organic chemical raw materials. Phenol is an important intermediate for the preparation of phenolic resins, epoxy resins, and polycarbonates, and also used in the fields of medicine, pesticides, and dyes. The global demand has reached 11 million tons/year, and it is expected that the consumption of phenol will increase at an average annual rate of 2.5% in the future. Cyclohexanone is mainly used to prepare important monomer raw materials such as caprolactam and adipic acid, and then applied to the production of polymer materials such as nylon and polyurethane. At the same time, cyclohexanone can also be used as an important industrial solvents, the global demand has reached 4.9 million tons/year.

At present, the most important industrial production method of phenol is the oxidative decomposition method of cumene (Hock method), which accounts for over 90% of the total production capacity of phenol. The first step of the cumene process is to alkylate benzene with propylene to produce cumene. The supply of propylene raw materials will seriously restrict the overall economic efficiency of the production equipment. In addition, the cumene method will co-produce a large amount of acetone, but there is a surplus of acetone, and the added value of the product is low. The cyclohexylbenzene method provides an alternative process route for the production of phenol, and the co-product of the reaction is cyclohexanone with high added value, which avoids the problem of excess acetone by-product in the cumene method. The previously developed cyclohexylbenzene method to produce phenol and cyclohexanone mainly includes three reaction processes: the one-step process of benzene hydroalkylation to cyclohexylbenzene (CHB), and the oxidation of cyclohexylbenzene to cyclohexylbenzene-1-hydroperoxide (1-CHBHP), cyclohexylbenzene hydroperoxide acid decomposition to produce phenol and cyclohexanone (formula A). 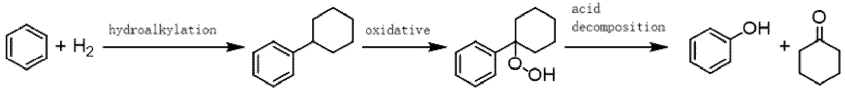 Formula A: The reaction route of generating phenol and cyclohexanone by cyclohexylbenzene method

Domestic and foreign chemical companies and research institutions have made certain progress in the research and development of technologies related to the cyclohexylbenzene method. Among them, ExxonMobil Corporation has applied for a series of patents (for example: CN105829273B, CN105793222A, CN105461535A, CN103664534A, CN102083777B, CN102015604A.), has reported a kind of cyclohexylbenzene process, wherein cyclohexylbenzene (CHB) oxidation process and cyclohexylbenzene-1-hydroperoxide (1-CHBHP) acidolysis process includes: using oxygen-containing gases such as oxygen, pure air or other oxygen-containing mixture as oxidant, N-Hydroxyphthalimide(NHPI) as catalyst, the reaction temperature is 90-130°C, and the reaction pressure is 50-10000kPa. The acidolysis reaction uses sulfuric acid as a catalyst, the reaction temperature is 40-120°C, and the reaction pressure is 100-1000kPa. However, the process is relatively long, and the accumulation of alkyl peroxides in the peroxidation reaction will also pose a potential safety risk. Besides generating phenol and cyclohexanone, the acid decomposition reaction of cyclohexylbenzene-1-hydroperoxide (1-CHBHP) also has some other by-products, which further reduces the overall selectivity of the process and increases the difficulty of subsequent product separation. The sulfuric acid catalyst in the acidolysis reaction will cause equipment corrosion and the post-treatment of the reaction will produce a large amount of phenolic wastewater.

The production of cyclohexanone mainly includes the oxidative decomposition method of cyclohexane and the hydration-dehydrogenation method of cyclohexene, wherein the oxidative decomposition method of cyclohexane includes four reaction processes: 1. hydrogenation of benzene to cyclohexane; 2. air oxidation of cyclohexane to produce cyclohexane hydroperoxide; 3. Decomposition of cyclohexane hydroperoxide under the action of catalyst to produce cyclohexanone and cyclohexanol; 4. dehydrogenation of cyclohexanol to produce cyclohexanone. However, this method has disadvantages such as long process flow, low conversion rate per pass (3-5%), poor product selectivity, and large discharge of three wastes. The cyclohexene hydration-dehydrogenation method is that cyclohexene and water undergo a hydration reaction under the action of a catalyst to generate cyclohexanol, and cyclohexanol is catalytically dehydrogenated and refined to obtain cyclohexanone products. However, this method has problems such as the high cost of cyclohexene, low conversion rate per pass, and long process flow.

Due to its advantages such as low cost, abundant reserves, green, non-toxic, and renewable, visible light-mediated photocatalytic strategies have become a powerful synthesis platform in recent years, which can achieve efficient activation of organic molecules under mild conditions, and then realized some previously difficult selective chemical transformations (Capaldo, L.; Ravelli, D.; Fagnoni, M. Chem. Rev. 2021. doi.org/10.1021/acs.chemrev.1c00263; Marzo, L.; Pagire, S. K.; Reiser, O.; König, B. Angew. Chem. Int. Ed. 2018, 57, 10034; Shaw, M. H.; Twilton, J.; MacMillan, D. W. C. J. Org. Chem. 2016, 81, 6898.).

### SUMMARY OF THE INVENTION

In order to overcome the defects in the prior art, the purpose of the present invention is to provide a green, light-mediated method for the one-step synthesis of phenol and cyclohexanone from cyclohexylbenzene using oxygen as an oxidant. This method features a simple process, mild reaction conditions, low cost, ease of operation, easy to control , and is suitable for industrial production. The present invention allows for the one-step synthesis of phenol and cyclohexanone from cyclohexylbenzene, avoiding the need for cyclohexylbenzene (CHB) to be oxidized to cyclohexylbenzene-1-hydroperoxide (1-CHBHP), the oxidation reaction product mixture is treated and then decomposed under acidic conditions to obtain phenol and cyclohexanone in the prior art.

The invention provides a light-mediated method for the one-step preparation of phenol and cyclohexanone from cyclohexylbenzene, catalyzed by a hydrogen bromide solution and using oxygen as the oxidant. Under the conditions of light irradiation and one atmospheric of oxygen pressure, cyclohexylbenzene is used as the raw material, an inexpensive and readily available hydrogen bromide solution as the catalyst, and oxygen as the oxidant in an organic solvent to directly produce phenol and cyclohexanone. The reaction process is as shown in the reaction formula (1): 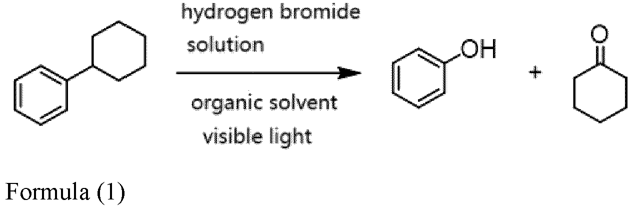 Wherein,
In the method of the present invention, the light is visible light.

In the method of the present invention, the light source used in the reaction is preferably a white LED lamp, and the power is preferably 60W.

In the method of the present invention, the catalyst is preferably an aqueous hydrogen bromide solution, and the mass fraction of the hydrogen bromide is preferably 40%.

In the method of the present invention, phenol can also be added as a reaction additive.

In the method of the present invention, the additive is phenol or phenol with electron-withdrawing substituents, including phenol, fluorine-substituted phenol, chlorine-substituted phenol, bromine-substituted phenol, nitro-substituted phenol, trifluoromethyl-substituted phenol, acetyl-substituted phenol, tert-butyl-substituted phenol, methyl-substituted phenol, cyano-substituted phenol, etc.; preferably, phenol, *p*-fluorophenol, *p*-chlorophenol, *p*-bromophenol, *p*-nitrophenol , *p*-trifluoromethylphenol, *p*-acetylphenol, *p*-cyanophenol; more preferably, it is phenol.

In the method of the present invention, the mol ratio of cyclohexylbenzene, hydrogen bromide, additive is 100:(0.1 ∼ 100):(0 ∼ 100), when the reaction scale is 1mmol, the molar ratio of cyclohexylbenzene, hydrogen bromide, additive is preferably 100:(20-30):(0-7.5), and the reaction time is preferably 9-24 hours.

In the method of the present invention, the mol ratio of cyclohexylbenzene, hydrogen bromide, additive is 100:(0.1 ∼ 100):(0 ∼ 100), when the reaction scale is 36mmol, the molar ratio of cyclohexylbenzene, hydrogen bromide, additive is preferably 100:(5-15):(0-3), and the reaction time is preferably 6-15 hours.

In the method of the present invention, the organic solvent is a mixture of one or more of 1,2-dichloroethane, 1,1-dibromomethane, dichloromethane, chloroform, acetonitrile, ethyl acetate, and acetone; preferably, the organic solvent is acetone.

In the method of the present invention, the source of the oxygen is pure oxygen or oxygen from the air.

In the method of the present invention, the reaction is preferably carried out at room temperature.

The beneficial effects of the present invention include providing a green, light-mediated method for the one-step synthesis of phenol and cyclohexanone from cyclohexylbenzene using oxygen as the oxidant. This method features a simple process, mild reaction conditions, low cost, ease of operation, easy to control, and suitable for industrial production.

### PREFERRED EMBODIMENTS OF THE INVENTION

The following examples are given to further illustrating the specific solutions of the present invention. The process, conditions, experimental methods, and so on for implementing the present invention are all general knowledge and common knowledge in the field except for the contents specifically mentioned below, and the present invention has no special limitation.

### Examples 1-4

Cyclohexylbenzene (CHB, 0.17 mL, 1.0 mmol), hydrobromic acid solution (40 wt.% aqueous solution, 45 µL), phenolic additive (0.01 mmol) and acetone (90 µL) were sequentially added to a reaction flask. The reaction flask was sealed with a flap plug, connected to an oxygen balloon, and subjected to irradiation under a white LED lamp (60 W) for 24 hours, with a fan used to maintain the reaction temperature at room temperature. Upon completion of the reaction, dibromomethane (35 µL, 0.5 mmol) and deuterated chloroform (0.5 mL) were added to the reaction solution. After mixing, 100 µL of the solution was transferred to an NMR tube, diluted with 0.5 mL of deuterated chloroform, and analyzed *via* NMR spectroscopy. The results are shown in Table 1.

**Table 1**

| Entry | Example 1 | Example 2 | Example 3 | Example 4 |
|---|---|---|---|---|
| Phenolic additive | Phenol | *p*-Nitrophenol | *p*-Cyanophenol | *p*-Cresol |
| Yield of phenol (%) | 33.9 | 29.7 | 29.5 | 6.4 |
| Yield of cyclohexanone (%) | 31.6 | 26.9 | 29.2 | 5.7 |
| Conversion of cyclohexylbenzene (%) | 38.3 | 34.6 | 33.1 | 12.2 |

### Examples 5-7

Cyclohexylbenzene (CHB, 6.0 mL), hydrobromic acid solution (40 wt.% aqueous solution, 0.6 mL), phenol (X mg), and acetone (1.0 mL) were sequentially added to a reaction flask. The flask was purged with oxygen, connected to an oxygen balloon, and irradiated under a white LED lamp (60 W) for 12 hours, with a fan used to maintain the reaction temperature at room temperature. Upon completion of the reaction, 0.2 mL of the reaction mixture was taken, and dibromomethane (35 µL, 0.5 mmol) and deuterated chloroform (0.5 mL) were added. After mixing, 100 µL of the mixture was transferred to an NMR tube, diluted with deuterated chloroform (0.5 mL), and analyzed *via* NMR spectroscopy. The results are shown in Table 2.

**Table 2**

| Entry | Example 5 | Example 6 | Example 7 |
|---|---|---|---|
| X (mg) | 88.5 | 44.3 | 0 |
| Yield of phenol (%) | 23.9 | 19.3 | 5.8 |
| Yield of cyclohexanone (%) | 23.4 | 13.4 | 3.4 |
| Conversion of cyclohexylbenzene (%) | 24.4 | 21.3 | - |

### Examples 8-9

Cyclohexylbenzene (CHB, 6.0 mL), hydrobromic acid solution (40 wt.% aqueous solution, X mL), phenol (88.5 mg), and acetone (1.0 mL) were sequentially added to a reaction flask. The flask was purged with oxygen, connected to an oxygen balloon, and irradiated under a white LED lamp (60 W) for 12 hours, with a fan used to maintain the reaction temperature at room temperature. Upon completion of the reaction, 0.2 mL of the reaction mixture was taken, and dibromomethane (35 µL, 0.5 mmol) and deuterated chloroform (0.5 mL) were added. After mixing, 100 µL of the mixture was transferred to an NMR tube, diluted with deuterated chloroform (0.5 mL), and analyzed *via* NMR spectroscopy. The results are shown in Table 3.

**Table 3**

| Entry | Example 8 | Example 9 |
|---|---|---|
| Hydrobromic acid solution (X mL) | 0.3 | 1.2 |
| Yield of phenol (%) | 16.8 | 3.1 |
| Yield of cyclohexanone (%) | 12.2 | 2.0 |
| Conversion of Cyclohexylbenzene (%) | 20.3 | - |

### Examples 10-13

Cyclohexylbenzene (CHB, 6.0 mL), hydrobromic acid solution (40 wt.% aqueous solution, 0.6 mL), phenol (88.5 mg), and acetone (1.0 mL) were sequentially added to a reaction flask. The flask was purged with oxygen, connected to an oxygen balloon, and irradiated under a white LED lamp (60 W) for a specified period, with a fan used to maintain the reaction temperature at room temperature. Upon completion of the reaction, 0.2 mL of the reaction mixture was taken, and dibromomethane (35 µL, 0.5 mmol) and deuterated chloroform (0.5 mL) were added. After mixing, 100 µL of the mixture was transferred to an NMR tube, diluted with deuterated chloroform (0.5 mL), and analyzed *via* NMR spectroscopy. The results are shown in Table 4.

**Table 4**

| Entry | Example 10 | Example 11 | Example 12 | Example 13 |
|---|---|---|---|---|
| Reaction time (h) | 6 | 9 | 15 | 24 |
| Yield of phenol (%) | 10.3 | 21.8 | 23.8 | 24.6 |
| Yield of cyclohexanone (%) | 9.6 | 20.0 | 22.3 | 18.0 |
| Conversion of Cyclohexylbenzene (%) | 11.5 | 22.1 | 28.8 | 35.8 |

### Examples 14-16

Cyclohexylbenzene (CHB, 6.0 mL), hydrobromic acid solution (40 wt.% aqueous solution, 0.6 mL), phenol (88.5 mg), and acetone (1.0 mL) were sequentially added to a reaction flask. The flask was purged with oxygen, connected to an oxygen balloon, and irradiated under a white LED lamp (60 W) at a specific temperature for 12 hours. Upon completion of the reaction, 0.2 mL of the reaction mixture was taken, and dibromomethane (35 µL, 0.5 mmol) and deuterated chloroform (0.5 mL) were added. After mixing, 100 µL of the mixture was transferred to an NMR tube, diluted with deuterated chloroform (0.5 mL), and analyzed *via* NMR spectroscopy. The results are shown in Table 5.

**Table 5**

| Entry | Example 14 | Example 15 | Example 16 |
|---|---|---|---|
| Reaction temperature(°C) | 30 | 40 | 50 |
| Yield of phenol (%) | 22.7 | 23.6 | 21.9 |
| Yield of cyclohexanone (%) | 21.8 | 19.7 | 15.4 |
| Conversion of Cyclohexylbenzene (%) | 24.1 | 26.1 | 26.9 |

### Examples 17-18

Cyclohexylbenzene (CHB, 6.0 mL), hydrobromic acid solution (40 wt.% aqueous solution, 0.6 mL), phenol (88.5 mg), and acetone (1.0 mL) were sequentially added to a reaction flask. The flask was purged with oxygen, connected to an oxygen balloon, and irradiated under a white LED lamp for 12 hours, with a fan used to maintain the reaction temperature at room temperature. Upon completion of the reaction, 0.2 mL of the reaction mixture was taken, and dibromomethane (35 µL, 0.5 mmol) and deuterated chloroform (0.5 mL) were added. After mixing, 100 µL of the mixture was transferred to an NMR tube, diluted with deuterated chloroform (0.5 mL), and analyzed *via* NMR spectroscopy. The results are shown in Table 6.

**Table 6**

| Entry | Example 17 | Example 18 |
|---|---|---|
| LED light power (W) | 30 | 90 |
| Yield of phenol (%) | 16.2 | 20.9 |
| Yield of cyclohexanone (%) | 16.0 | 20.1 |
| Conversion of Cyclohexylbenzene (%) | 18.4 | 22.9 |

### Examples 19-23

Cyclohexylbenzene (CHB, 6.0 mL), hydrobromic acid solution (40 wt.% aqueous solution, 0.6 mL), phenol (88.5 mg), and solvent (1.0 mL) were sequentially added to a reaction flask. The flask was purged with oxygen, connected to an oxygen balloon, and irradiated under a white LED lamp (90 W) for 12 hours, with a fan used to maintain the reaction temperature at room temperature. Upon completion of the reaction, 0.2 mL of the reaction mixture was taken, and dibromomethane (35 µL, 0.5 mmol) and deuterated chloroform (0.5 mL) were added. After mixing, 100 µL of the mixture was transferred to an NMR tube, diluted with deuterated chloroform (0.5 mL), and analyzed *via* NMR spectroscopy. The results are shown in Table 7.

**Table 7**

| Entry | Example 19 | Example 20 | Example 21 | Example 22 | Example 23 |
|---|---|---|---|---|---|
| Reaction solvent(mL) | Acetone (0.5) | Ethyl acetate (1.0) | 1,2-Dichlor oethane (1.0) | Acetone/ Dichlorome thane = 1:1 (1.0) | No solvent |
| Yield of phenol (%) | 22.2 | 16.3 | 13.9 | 16.8 | 7.6 |
| Yield of cyclohexanone (%) | 14.8 | 8.3 | 8.9 | 10.7 | 5.8 |
| Conversion of Cyclohexylbenz ene (%) | 23.2 | 27.1 | 16.4 | 30.0 | 8.6 |

### Examples 24-27

Cyclohexylbenzene (CHB, 6.0 mL), hydrobromic acid solution (40 wt.% aqueous solution, 0.6 mL), phenol (88.5 mg), and acetone (1.0 mL) were sequentially added to a reaction flask. The flask was purged with oxygen, connected to an oxygen balloon, and irradiated under different light sources (60 W) for 6 hours, with a fan used to maintain the reaction temperature at room temperature. Upon completion of the reaction, 0.2 mL of the reaction mixture was taken, and dibromomethane (35 µL, 0.5 mmol) and deuterated chloroform (0.5 mL) were added. After mixing, 100 µL of the mixture was transferred to an NMR tube, diluted with deuterated chloroform (0.5 mL), and analyzed *via* NMR spectroscopy. The results are shown in Table 8.

**Table 8**

| Entry | Example 24 | Example 25 | Example 26 | Example 27 |
|---|---|---|---|---|
| Light sources | Blue LED | Purple LED | Fluorescent lamp CFL | No light source (dark reaction) |
| Yield of phenol (%) | 22.2 | 14.0 | 3.7 | 0 |
| Yield of cyclohexanone (%) | 16.3 | 13.3 | 2.9 | 0 |
| Conversion of Cyclohexylbenzene (%) | 26.1 | 29.8 | - | 0 |

### Examples 28-32

Cyclohexylbenzene (CHB, 6.0 mL), lithium bromide (347.4 mg), an acid additive (4 mmol), phenol (88.5 mg), and acetone (1.0 mL) were sequentially added to a reaction flask. The flask was purged with oxygen, connected to an oxygen balloon, and irradiated under a white LED lamp (60 W) for 12 hours, with a fan used to maintain the reaction temperature at room temperature. Upon completion of the reaction, 0.2 mL of the reaction mixture was taken, and dibromomethane (35 µL, 0.5 mmol) and deuterated chloroform (0.5 mL) were added. After mixing, 100 µL of the mixture was transferred to an NMR tube, diluted with deuterated chloroform (0.5 mL), and analyzed *via* NMR spectroscopy. The results are shown in Table 9.

**Table 9**

| Entry | Example 28 | Example 29 | Example 30 | Example 31 | Example 32 |
|---|---|---|---|---|---|
| Acid additives | Concentrate d sulfuric acid | *p*-Toluenesu lfonic acid | Concentrate d hydrochloric acid | Acetic acid | None |
| Yield of phenol (%) | 8.1 | 9.0 | 11.6 | 0 | 0 |
| Yield of cyclohexanone (%) | 4.4 | 6.3 | 8.7 | 0 | 0 |
| Conversion of Cyclohexylben zene (%) | 9.6 | 11.2 | 18.8 | 0 | 0 |

### Examples 33-36

Cyclohexylbenzene (CHB, 6.0 mL), concentrated hydrochloric acid (0.34 mL), a bromide (4 mmol), phenol (88.5 mg), and acetone (1.0 mL) were sequentially added to a reaction flask. The flask was purged with oxygen, connected to an oxygen balloon, and irradiated under a white LED lamp (60 W) for 12 hours, with a fan used to maintain the reaction temperature at room temperature. Upon completion of the reaction, 0.2 mL of the reaction mixture was taken, and dibromomethane (35 µL, 0.5 mmol) and deuterated chloroform (0.5 mL) were added. After mixing, 100 µL of the mixture was transferred to an NMR tube, diluted with deuterated chloroform (0.5 mL), and analyzed *via* NMR spectroscopy. The results are shown in Table 10.

**Table 10**

| Entry | Example 33 | Example 34 | Example 35 | Example 36 |
|---|---|---|---|---|
| Bromides | Potassium bromide | Magnesium bromide | Tetrabutylammoni um bromide | None |
| Yield of phenol (%) | 19.9 | 16.4 | 0 | 0 |
| Yield of cyclohexanone (%) | 14.9 | 11.6 | 0 | 0 |
| Conversion of Cyclohexylbenzene (%) | 20.8 | 20.0 | 0 | 0 |

### Examples 37-38

Cyclohexylbenzene (CHB, 6.0 mL), hydrobromic acid solution (40 wt.% aqueous solution, 0.6 mL), phenol (88.5 mg), and acetone (1.0 mL) were sequentially added to a reaction flask. The flask was then placed under different atmospheric conditions and irradiated with a white LED lamp (60 W) for 12 hours, with a fan used to maintain the reaction temperature at room temperature. Upon completion of the reaction, 0.2 mL of the reaction mixture was taken, and dibromomethane (35 µL, 0.5 mmol) and deuterated chloroform (0.5 mL) were added. After mixing, 100 µL of the mixture was transferred to an NMR tube, diluted with deuterated chloroform (0.5 mL), and analyzed *via* NMR spectroscopy. The results are shown in Table 11.

**Table 11**

| Entry | Example 37 | Example 38 |
|---|---|---|
| Atmosphere conditions | Air balloon | Air bubbling (100mL/min) |
| Yield of phenol (%) | 4.9 | 2.3 |
| Yield of cyclohexanone (%) | 4.1 | 1.7 |
| Conversion of Cyclohexylbenzene (%) | - | - |

### Example 39

Cyclohexylbenzene (CHB, 6.0 mL), hydrobromic acid solution (40 wt.% aqueous solution, 0.6 mL), phenol (88.5 mg), and acetone (1.0 mL) were sequentially added to a reaction flask. The flask was purged with oxygen, connected to an oxygen balloon, and placed in a 50 °C oil bath under dark conditions for 12 hours. Upon completion of the reaction, 0.2 mL of the reaction mixture was taken, and dibromomethane (35 µL, 0.5 mmol) and deuterated chloroform (0.5 mL) were added. After mixing, 100 µL of the mixture was transferred to an NMR tube, diluted with deuterated chloroform (0.5 mL), and analyzed *via* NMR spectroscopy. The yield of phenol was 4.2%, the yield of cyclohexanone was 4.0%, and the conversion of cyclohexylbenzene was 7.3%.

### Example 40

Cumene (6.0 mL), hydrobromic acid solution (40 wt.% aqueous solution, 0.6 mL), phenol (88.5 mg), and acetone (1.0 mL) were sequentially added to a reaction flask. The flask was purged with air, connected to an air balloon, and irradiated with a white LED lamp (60 W) for 12 hours, with a fan used to maintain the reaction temperature at room temperature. Upon completion of the reaction, 0.2 mL of the reaction mixture was taken, and dibromomethane (35 µL, 0.5 mmol) and deuterated chloroform (0.5 mL) were added. After mixing, 100 µL of the mixture was transferred to an NMR tube, diluted with deuterated chloroform (0.5 mL), and analyzed *via* NMR spectroscopy. The yield of phenol was 19.6%, and the conversion of cumene was 26.6%.

The protection content of the present invention is not limited to the above embodiments. Without departing from the spirit and scope of the concept of the present invention, changes and advantages conceivable by those skilled in the art are all included in the present invention, and the appended claims are the protection scope.

## Claims

1. A light-mediated one-step method for preparing phenol and cyclohexanone from cyclohexylbenzene, wherein, the process realizes directly oxidized carbon-carbon bond cleavage of cyclohexylbenzene and generates phenol and cyclohexanone in organic solvent with cyclohexylbenzene as raw material, using a hydrogen bromide solution as a catalyst and oxygen as an oxidizing agent under light irradiation; the reaction process is shown in the reaction formula (1):

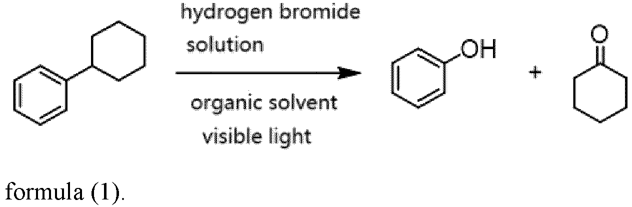

2. The method according to claim 1, wherein, the catalyst is an aqueous hydrogen bromide solution, wherein the mass fraction of the hydrogen bromide is 40%.

3. The method according to claim 1, wherein, the mol ratio of the hydrogen bromide to cyclohexylbenzene is (0.1-100): 100.

4. The method according to claim 1, wherein, the light is visible light.

5. The method according to claim 1, wherein, the organic solvent is a mixture of one or more of 1,2-dichloroethane, 1,1-dibromomethane, dichloromethane, chloroform, acetonitrile, ethyl acetate, and acetone.

6. The method according to claim 1, wherein, the source of the oxygen is pure oxygen or oxygen from the air.

7. The method according to claim 1, wherein, phenol can be added as a reaction additive, wherein the additive is phenol or substituted phenol, including phenol, fluorine-substituted phenol, chlorine-substituted phenol, bromine-substituted phenol, nitro-substituted phenol, trifluoromethyl-substituted phenol, acetyl-substituted phenol, tert-butyl-substituted phenol, methyl-substituted phenol, and cyano-substituted phenol.

8. The method according to claim 7, wherein, the mol ratio of the additive to cyclohexylbenzene is (0-100): 100.

9. The method according to claim 1, wherein, the reaction time is 6-24h.

10. Application of the method as described in any one of claims 1-9 in the one-step preparation of phenol and cyclohexanone from cyclohexylbenzene.
